# EUROPÄISCHE PATENTSCHRIFT

(11) **EP 0 846 690 B1**
(45) Veröffentlichungstag und Bekanntmachung des Hinweises auf die Patenterteilung: **23.01.2002**
(21) Anmeldenummer: 97118132.6
(22) Anmeldetag: 18.10.1997
(51) Int. Cl.: C07D 251/64, C23F 11/10, C23F 11/16

(54) **Melamin-polycarbonsäureamide und ihre Verwendung als Korrosionsschutzmittel**
Melamin polycarboxylic amides and their use as corrosion inhibitor
Amides d'acides polycarboxyliques de mélamine et leur utilisation comme inhibiteur de corrosion

(30) Priorität: 26.11.1996 DE 19648843
(43) Veröffentlichungstag der Anmeldung: 10.06.1998
(73) Patentinhaber: ZTS-Chemie GmbH, 63505 Langenselbold (DE)
(72) Erfinder: Graichen, Stefan, 63546 Hammersbach (DE)
(74) Vertreter: Meyer-Dulheuer, Karl-Hermann, Dr.

(56) Entgegenhaltungen:
- EP-A- 0 041 039
- EP-A- 0 046 139
- EP-A- 0 129 506
- EP-A- 0 398 284
- EP-A- 0 511 163
- EP-A- 0 541 966
- EP-A- 0 553 962
- EP-A- 0 554 023
- EP-A- 0 565 774
- US-A- 2 485 309

## Beschreibung

Gegenstand der Erfindung sind Melamin-polycarbonsäureamide und ihre Verwendung zur Inhibierung der Korrosion von Eisen oder eisenhaltigen Metallen, die in Kontakt mit wäßrigen Systemen stehen.

Es ist bekannt, daß erhebliche Anstrengungen unternommen werden, um die Korrosion von Metallen zu vermindern. So ist in der europäischen Patentschrift 46 139 die verwendung von Triazincarbonsäuren als Korrosionsinhibitoren für wäßrige Systeme, die in Kontakt mit Eisen oder eisenhaltigen Metallen stehen, vorgeschlagen worden. Aus der europäischen Patentschrift 511 163 sind fließfähige wäßrige Dispersionen bekannt, die einen festen Polycarbonsäuretriazin-Korrosionsinhibitor enthalten. Weiterhin ist aus US-A-4 402 907 und der EP-A-129 506 bekannt, daß bestimmte heterozyklische Polycarbonsäuren als Korrosionsinhibitoren für wäßrige Systeme, die mit Metallen in Kontakt stehen, gut geeignet sind. Sie können in wäßrigen Systemen, zum Beispiel in Kühlwassersystemen, in Dampferzeugungsanlagen, in Metallbearbeitungsmitteln und wäßrigen Hydraulikflüssigkeiten verwendet werden. Da die meisten Polycarbonsäuren in Wasser nur eine geringe Löslichkeit haben, verwendet man die Polycarbonsäuren in Form ihrer wasserlöslichen Salze, d.h., man neutralisiert sie vor dem Gebrauch oder man setzt sie einem basischen Wassersystem zu. Lager- und Handelsform sind jedoch im allgemeinen die freien Polycarbonsäuren.

Die freien Polycarbonsäuren sind im allgemeinen feste Stoffe. Bei ihrer Herstellung werden sie meist durch Filtration aus einer wässrigen Phase isoliert. Das filtrierte Produkt wird üblicherweise mit Wasser gewaschen und dann einer Trocknung unterzogen. Um die für die Trocknung benötigte Energie zu sparen, hat man neuerdings für die Verwendung in wässrigen Systemen den feuchten Filterkuchen, der etwa 50% Wasser enthält, als Handelsform angeboten. Der feuchte Filterkuchen hat jedoch den Nachteil, dass er nicht fließfähig ist. Er kann nicht geschüttet oder gegossen werden, sondern wird mit der Schaufel manuell dosiert oder umgefüllt. Deshalb ist auch schon vorgeschlagen worden, Polycarbonsäuren in Form von hochprozentigen wässrigen Dispersionen einzusetzen.

Auch auf der Basis von Melamin entwickelte Korrosionsinhibitoren sind bereits bekannt. So werden in der US-A-2 485 309 Methylolmelamin-Kondensationsprodukte beschrieben, die aber den hohen Anforderungen, welche heute an ein Korrosionsschutzmittel gestellt werden, keinesfalls genügen.

Aus der EP-A-0 553 962 ist eine Korrosions-Inhibitor-Zusammensetzung bekannt, die neben einem carboxylgruppenhaltigen Korrosionsinhibitor, z. B. einer Triazincarbonsäure, noch eine Aminobenzoesäure, ein Alkanolamin und ein Borat enthält. Gute Ergebnisse werden aber nicht erhalten, wenn die Triazincarbonsäure als alleiniger Korrosionsschutz eingesetzt wird.

Aus der EP-A-0 398 284 ist ein Frostschutzmittel bekannt, das als Korrosionsinhibitor eine Triazincarbonsäure zusammen mit weiteren Additiven enthält. Melamin-polycarbonsäureamide werden dabei nicht verwendet.

Aus der EP-A-0 041 039 ist die Verwendung von Triazincarbonsäuren bekannt, die Mercaptogruppen enthalten und als Korrosionsinhibitoren eingesetzt werden können.

Die EP-A-0 554 023 beschreibt ein antikorrosives Beschichtungsmittel, das Salze von Carbonsäuren enthält. Melamin-polycarbonsäureamide werden dort nicht erwähnt.

Die EP-A-0 541 966 und die EP-A-0 565 774 beschreiben Verfahren zur Herstellung von Säureamidderivaten durch eine Reaktion von Haloaminen und Säurehalogeniden. Auf diesem Weg lassen sich auch spezielle Melaminderivate herstellen, die reaktive Gruppen aufweisen und für weitere Umsetzungen geeignet sind. Der Korrosionsschutz wird dort nicht angesprochen.

Es wurde nun gefunden, dass neuartige Melamin-polycarbonsäureamide und ihre wasserlöslichen Salze ausgezeichnete Korrosionsinhibitoren in wässrigen Systemen sind und sich zum Einsatz in Wasserkreisläufen, in wässrigen Maschinenflüssigkeiten, Gefrierschutzflüssigkeiten, hydraulischen Flüssigkeiten oder wässrigen Anstrichmitteln hervorragend eignen.

Gegenstand der Erfindung sind deshalb Melamin-polycarbonsäureamide der Formel 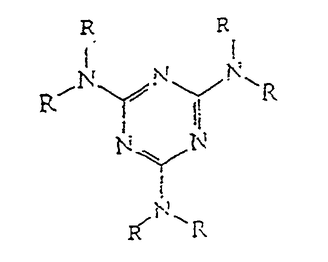 in der mindestens einer der Reste R ein Substituent der Struktur II 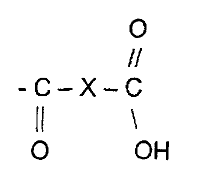 ist, dessen Carboxylgruppe mit einem ein- oder mehrwertigen Alkohol mit bis zu 6 Kohlenstoffatomen teilweise oder vollständig verestert oder mit einem weiteren Melaminrest kondensiert sein kann und X ein geradkettiger oder verzweigter Alkylenrest mit ein bis zwölf Kohlenstoffatomen sein kann, der auch eine Cyclopentylen-, Cyclohexylen- oder Phenlengruppe enthalten oder

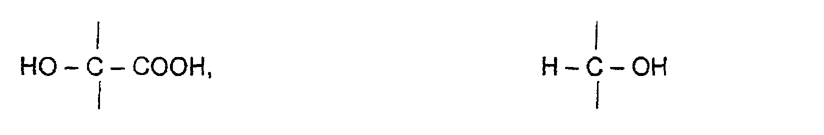

-[-CH₂-CH₂-O-]-ₙ - (CHOH)ₙ-(CH₂)ₘ-

oder 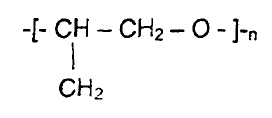 bedeuten kann, wobei m und n Werte zwischen 1 und 20 annehmen können und die anderen Reste R Wasserstoff, eine Alkylgruppe oder eine Acylgruppe mit bis zu 12 Kohlenwasserstoffatomen sein können, sowie ihre Alkali-, Amin- oder Ammoniumsalze.

In den erfindungsgemäßen Melamin-polycarbonsäureamiden kann sich der Rest R von einer gesättigten, geradkettigen oder verzweigten Dicarbonsäure, z. B. der Butandisäure, der Pentandisäure, der Hexandisäure, der Heptandisäure, der Nonandisäure und bevorzugt der Decandisäure ableiten. Die Kohlenstoffkette dieser Dicarbonsäuren kann auch einen Cyclopentylen-, Cyclohexylen- oder Phenylenrest enthalten. Auch Zitronensäure oder eine Ethylenoxid- oder Propylenoxid-Einheiten enthaltende Dicarbonsäure kann mit Melamin zu den wertvollen, erfindungsgemäßen Korrosionsschutzmitteln umgesetzt werden.

Besonders bevorzugt sind Melamin-polycarbonsäureamide, bei denen alle drei Aminogruppen des Melamins acyliert sind. Es können jedoch auch Melaminderivate eingesetzt werden, bei denen nur eine oder zwei Aminogruppen acyliert sind. Die zur Reaktion mit dem Melamin verwendeten Di- oder Tricarbonsäuren werden im allgemeinen in solchen Mengenverhältnissen mit dem Melamin umgesetzt, dass wenigstens jeweils eine Carboxylgruppe der Di- oder Tricarbonsäure frei erhalten bleibt. Die Wirksamkeit der erfindungsgemäße Melamin-polycarbonsäureamide als Korrosionsschutzmittel wird jedoch nicht eingeschränkt, wenn durch Anwendung eines Überschusses an Melamin auch ein Teil der zweiten Carbonsäure der eingesetzten Dicarbonsäuren eine Amidbindung mit einem weiteren Melaminmolekül eingeht. Es sollten jedoch mindestens 80%, vorzugsweise mehr als 90% der Di- oder Tricarbonsäurestruktureinheiten noch eine freie Carbonsäure aufweisen, damit sich die erfindungsgemäßen Melamin-polycarbonsäureamide im alkalischen Medium unter Salzbildung in Wasser lösen können.

Die Herstellung der Melamin-polycarbonsäureamide erfolgt dadurch, daß die Di- oder Tricarbonsäure oder das entsprechende Dicarbonsäurehalogenid, das Dicarbonsäureanhydrid oder der Dicarbonsäureester in einem Reaktionsgefäß unter Anwendung von Hitze aufgeschmolzen und dann, ggf. auch unter Anwendung eines inerten Schutzgases, Melamin in die Schmelze eingerührt wird. Dabei wird im allgemeinen die Dicarbonsäure oder das Dicarbonsäurederivat in der dreifach molaren Menge, bezogen auf das Melamin, eingesetzt, wodurch dendritartige Strukturen entstehen können. Es ist jedoch auch durchaus möglich, größere Mengen Melamin zu verwenden, so daß nicht alle Aminogruppen des Melamins acyliert werden.

Die für die Reaktion eingesetzte Dicarbonsäure oder das Dicarbonsäurederivat wird vorzugsweise eine einheitliche chemische Verbindung sein. Es können jedoch auch Gemische verschiedener Dicarbonsäuren eingesetzt werden, sofern sie der oben angegebenen Definition für das Strukturelement II entsprechen. Vorteilhafte Ergebnisse werden auch erzielt, wenn der Dicarbonsäure bis zu 20 Gew.-% einer Monocarbonsäure mit bis zu 12 Kohlenstoffatomen zugesetzt werden. Eine gute antikorrosive Wirkung wurde auch mit Melamin-polycarbonsäureamiden erzielt, bei denen die entstandenen freien Carboxylgruppen ganz oder teilweise mit einem Mono- oder Dialkylamim oder mit einem Mono- oder Dialkanolamin mit jeweils bis zu 10 Kohlenstoffatomen unter Amidbildung umgesetzt wurden.

Bei der Reaktion wird im allgemeinen eine Temperatur zwischen 120 und 180°C, vorzugsweise zwischen 160 und 170°C eingehalten. Nach etwa 30 Minuten ist die Reaktion im allgemeinen beendet. Das Reaktionsprodukt kann dann nach dem Erkalten aus dem Reaktionsgefäß entnommen und in einer wäßrig alkalischen Lösung zum Korrosionsschutzmittel gelöst werden. Zur Lösung der erfingungsgemäßen Melamin-polycarbonsäureamide können alle üblichen alkalischen Mittel eingesetzt werden. Besonders bevorzugt ist die Salzbildung mit Triethanolamin.

Die Verbindungen der Formel I werden vorzugsweise in einer Menge von 0,001 bis 10 Gewichtsprozent, bezogen auf das wäßrige System, verwendet. Bei dem wäßrigen System kann es sich um Wasserkreisläufe, beispielsweise Kühlwasserkreisläufe, Kreisläufe von wäßrigen Maschinenflüssigkeiten wie beispielsweise Kühlflüssigkeiten beim Bohren, Malen, Fräsen, Drehen, Schneiden, Sägen, Schleifen, Gewindeschneiden oder beim Walzen oder Ziehen von Metallen handeln. Auch Gefrierschutzmittel oder hydraulische Flüssigkeiten auf Glykol-Wasserbasis sowie wäßrige Anstrichfarben, zum Beispiel Dispersionsfarben oder wäßrige Pulverlacke können mit den erfindungsgemäßen Melamin-polycarbonsäureamiden antikorrosiv ausgerüstet werden.

Die Verbindungen der Formel I können in den wäßrigen Systemen als alleiniger Zusatz oder in Kombination mit anderen Zusätzen verwendet werden. Beispiele für solche Co-Additive in Wasserkreisläufen sind bekannte Korrosionsinhibitoren wie Phosphonate, Phosphonocarbonsäuren oder Phosphinocarbonsäuren, N-Acylsarkosine, Imidazoline, Triethanolamin, Fettamine oder Mono- oder Polycarbonsäuren. Ganz besonders bewährt hat sich der Zusatz von Monocarbonsäuren, insbesondere der Isononansäure. Auch Kupfer-Passivatoren wie wasserlösliche Benztriazole, Methylen-bis-benztriazole oder 2-Mercaptobenzthiazole können zugesetzt werden. Weiterhin können Dispersionsmittel und Trägerstoffe, wie Poly(meth)acrylsäure und ihre Salze, hydrolysiertes Polyacrylnitril, Polyacrylamid und dessen Copolymere, Ligninsulfonsäure und deren Salze, Stärke und Stärkederivate, Cellulose, Alkylphosphonsäuren, 1-Aminoalkyl-1,1-diphosphonsäuren und ihre Salze, Polymaleinsäuren und andere Polycarbonsäuren oder Alkaliphosphate zugegeben werden.

Weitere Co-Additive können Fällungsmittel sein, wie Alkaliphosphate oder Alkalicarbonate, Sauerstoffabfänger wie Alkalisulfate oder Hydrazin, Komplexierungsmittel wie Nitrilotriessigsäure oder Ethylendiamin-tetraessigsäure und deren Salze, oder schaumverhütende Mittel wie Distearylsebacinsäurediamid, Distearyladipinsäurediamid oder Ethylenoxid- oder Propylenoxid-Kondensationsprodukte solcher Amide, sowie Fettalkohole und deren Ethylenoxid-Kondensationsprodukte.

Wäßrige Systeme, die als Maschinenflüssigkeiten verwendet werden, können ein wasserverdünnbares Schneid- oder Schleiföl sein, wie
a) wäßrige Konzentrate eines erfindungsgemäßen Melaminpolycarbonsäureamids mit oder ohne einen Antiverschleißzusatz, die dann in einer Verdünnung von 1 : 50 bis 1 : 100 als Schleifflüssigkeit verwendet werden können,
b) Polyglykole, die ein Melamin-polycarbonsäureamid, Biocide, Korrosionsinhibitoren und Antiverschleißmittel enthalten und die als Schneidflüssigkeit in einer Verdünnung von 1 : 20 bis 1 : 40 und als Schleifflüssigkeit in einer Verdünnung von 1 : 60 bis 1 : 80 verwendet werden können,
c) halbsynthetische Schneidöle auf ähnlicher Basis wie b), jedoch zusätzlich 10-25% eines Öls enthaltend sowie genügend Emulgator, um die Flüssigkeit beim Verdünnen transparent zu halten,
d) emulgierbare Mineralöl-Konzentrate, die außer dem Emulgator ein Melamin-polycarbonsäureamid, Antiverschleißmittel, Biocide, Antischaummittel enthalten können und die üblicherweise im Verhältnis 1 : 20 bis 1 : 50 mit Wasser verdünnt werden zu einer opaken Emulsion,
e) Produkte ähnlich d), jedoch weniger Öl und mehr Emulgator enthaltend, die bei einer Verdünnung von 1 : 50 bis 1 : 100 durchscheinende Emulsionen ergeben.

Auch in Gefrierschutzmittel oder Hydraulikflüssigkeiten können die Verbindungen der Formel I entweder allein oder in Kombination mit anderen Zusätzen verwendet werden. Zusätzlich können darin auch andere Korrosionsinhibitoren enthalten sein, wie
a) organische Säuren, deren Salze und Ester, zum Beispiel Benzoesäure, p-tert.Butylbenzoesäure, Dinatrium-sebacat, Triethanolamin-laurat, Isononansäure, das Triethanolaminsalz der p-Toluolsulfonamido-capronsäure, Natrium-N-lauroylsarcosinat oder Nonylphenoxyessigsäure;
b) stickstoffhaltige Substanzen, wie Fettsäurealkanolamide, Imidazoline, Oxazoline, Triazole oder anorganische Nitrite oder Nitrate;
c) phosphorhaltige Substanzen, beispielsweise Aminphosphate, Phosphonsäuren oder anorganische Phosphate, wie NaH₂PO₄;
d) schwefelhaltige Substanzen, beispielsweise Salze von Petroleumsulfonaten, oder heterocyclische verbindungen wie Natrium-mercaptobenzthiazol.

Die erfindungsgemäßen Melamin-polycarbonsäureamide können in einer wäßrig-alkalischen Lösung mit einem pH-Wert über 8,0 oder auch als fließfähige wäßrige Dispersionen eingesetzt werden. Als Dispergiermittel eignen sich alle oberflächenaktiven Verbindungen, insbesondere anionische und nichtionische Tenside. Derartige Dispersionen können durch verdickungsmittel stabilisiert werden, wobei man als Stabilisatoren vor allem modifizierte Polysaccharide vom Xanthan-, Alginat-, Guar- oder Cellulose-Typ verwendet. Dazu gehören auch Celluloseäther, wie Methylcellulose oder Carboxymethylcellulose und Heteropolysaccharide. Außer dem Dispergiermittel und dem Verdickungsmittel können die erfindungsgemäßen Dispersionen noch weitere Hilfsmittel enthalten, beispielsweise hydrotrope Mittel wie Harnstoff oder Natriumxylolsulfonat; Gefrierschutzmittel wie Ethylen- oder Propylenglykol, Diethylenglykol, Glycerin oder Sorbit; Biozide wie Chloracetamid, Formalin oder 1-2-Benzisothiazolin-3-on oder Komplexbildner. Zur Herstellung der Dispersionen geht man zweckmäßig vom festen Melamin-polycarbonsäureamid aus, setzt diesem das Dispergiermittel und das Verdickungsmittel sowie ggf. die gewünschte Menge Wasser und weitere Zusätze zu und rührt das Gemisch solange, bis eine fließfähige homogene Dispersion entstanden ist. Die so hergestellten Dispersionen sind bei Raumtemperatur sowie bei Temperaturen bis zu 40°C mehrere Monate stabil. Sie behalten ihre Fließfähigkeit und entmischen sich nicht. Das ist für die Lagerung und den Transport der Dispersionen eine wichtige Eigenschaft. Für die Verwendung der Dispersionen ist es von Vorteil, daß sie wie Flüssigkeiten gehandhabt werden können und sich sehr schnell in alkalisch-wäßrigen Systemen lösen.

Die Erfindung wird durch die folgenden Beispiele näher veranschaulicht:

### Beispiel 1: Bestimmung der Korrosionsschutzeigenschaften von Melaminpolycarbonsäureamide

Die Bestimmung der Korrosionsschutzeigenschaften von Melamin-polycarbonsäureamiden erfolgte gemäß der Vorschrift der DIN 51360 Teil II. Als Maß der Korrosion dienen dabei die Korrosionsabzeichnungen auf einem Rundfilterpapier, die bei der Einwirkung von künstlich korrosiv gemachtem Wasser auf Graugußspäne in Gegenwart der Melamin-polycarbonsäureamide entstehen. Der Versuch wird dabei in folgender Weise durchgeführt:
1. 1 Mol Melamin wird mit 1, 2 oder 3 Mol einer Di- oder Tricarbonsäure entweder unter Luftabschluß oder unter Schutzgas bei einer Temperatur von etwa 200°C aufgeschmolzen oder in Gegenwart eines hoch siedenden Lösungsmittels wie Dioxan erhitzt, bis die Kondensationsreaktion beendet ist. Dann wird die Schmelze abgekühlt, gebrochen und pulverisiert oder das verwendete Lösungsmittel abdestilliert. Das erhaltene Melamin-polycarbonsäureamid ist zu 8% bzw. zu 10% in einem Korrosionsschutzmittel enthalten, das außerdem noch aus 60% Triethanolamin rein (85%) und Leitungswasser besteht.
2. Entsprechend der Vorschrift der DIN 51360, Teil II wird nun in ein Becherglas Wasser mit einer Gesamthärte von 3,58 mmol, hergestellt aus CaCl₂ x 6H₂O und MgSo₄ x 7H₂O, mit 3% des oben genannten Korrosionsschutzmittels versetzt und mit dieser Lösung die in einer Petrischale auf Rundfilter verteilten Graugußspäne benetzt. Nach einer Verweilzeit von 2 Stunden bei Zimmertemperatur werden die Graugußspäne vom Rundfilter entfernt, das Rundfilter gespült und getrocknet und dann der Korrosionsgrad visuell entsprechend der Tabelle der DIN 51360, Teil II bestimmt.

Dabei wurden folgende Ergebnisse erhalten:

### Korrosionsgrade der Melamin-polycarbonsäureamide

| **Versuchs- Nr.** | **Melamin-polycarbonsäureamid aus** | **pH einer 3%- igen Lösung des Korrosionsschutzmittels** | **Korrosionsgrad (8%-ig)** | **Korrosionsgrad (10%-ig)** |
|---|---|---|---|---|
| 1 | 1 Mol Melamin + 3 Mol Decandisäure | 8,75 | 3 | 1 |
| 2 | 2 Mol Melamin + 5 Mol Decandisäure | 8,85 | 1-2 | 1 |
| 3 | 1 Mol Melamin + 3 Mol Terephthalsäure | 9,0 | 2-3 | 1 |
| 4 | Terephthalsäure (Vergleich) | 8,3 | 4 | 4 |
| 5 | 1 Mol Melamin + 2 Mol Glutarsäure | 8,75 | 2-3 | 2 |
| 6 | 2 Mol Melamin + 5 Mol Glutarsäure | 8,87 | 1-2 | 1 |
| 7 | 1 Mol Melamin + 1 Mol Adipinsäure | 8,2 | 2-3 | 1 |

Die Tabelle zeigt, daß gute antikorrosive Eigenschaften mit allen erfindungsgemäßen Melamin-polycarbonsäureamiden erhalten werden können, die sich noch verbessern, wenn das Melamin-polycarbonsäureamid im Korrosionsschutzmittel in einer höheren Konzentration, zum Beispiel zu 10%, enthalten ist. Bemerkenswert ist auch die verbesserte Korrosionsschutzwirkung bei den höher kondensierten Produkten der Versuchsnummern 2 und 6.

### Beispiel 2:

In einem mit Rührer versehenen Reaktionsbehälter werden 3 Mol Decandisäure vorgelegt und aufgeschmolzen. Bei einer Temperatur von etwa 170°C wird 1 Mol Melamin in die Schmelze eingerührt und 30 Minuten weiter erhitzt. Dann wird abgekühlt und der sich abscheidende Feststoff mit einer 85%-igen Lösung von Triethanolamin und Wasser in einer solchen Menge behandelt, daß eine Lösung entsteht, die 8% des erfindungsgemäßen Melamin-polycarbonsäureamids, 60% Triethanolamin (85%-ig) und 32% Wasser enthält.

Eine derartige Lösung kann in einer Menge von 3% einer Schleifflüssigkeit, einer Kühlerflüssigkeit oder einem Frostschutzmittel als Korrosionsinhibitor zugesetzt werden.

### Beispiel 3:

In einem mit Rührer versehenen Reaktionsbehälter werden 5 Mol Sebacinsäure und 2 Mol Melamin vermischt und aufgeschmolzen. Das sich beim Erkalten bildende feste Produkt wird in 65 g Triäthanolamin und Wasser zu 100 g gelöst. Mit Eisenspänen ergibt die Lösung einen Korrosionsgrad von 0 bis 1.

### Beispiel 4:

In einem mit Rührer versehenen Reaktionsbehälter werden 3 Mol Sebacinsäure und 1 Mol Melamin unter Erwärmen mit 50 g Wasser gemischt. Dabei entsteht eine homogene Suspension, die auf ein langsam laufendes Teflonband aufgebracht, getrocknet und dann bei 200°C schnell erhitzt wird.

5 g dieses Produktes werden mit 5 g Isononansäure und 65 g Triäthanolamin vermischt und mit Wasser auf 100 g aufgefüllt. Es ensteht ein Korrosionschutzmittel, das im Rosttest einen Korrosionsgrad von 0 bis 1 zeigt.

## Patentansprüche

1. Melamin-polycarbonsäureamid der Formel I 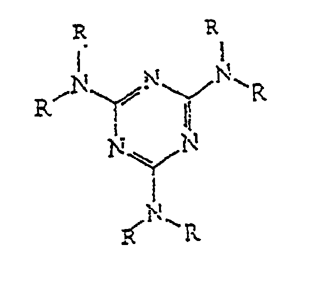 In der mindestens einer der Reste R ein Substituent der Struktur II 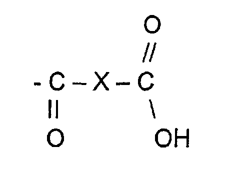 ist, dessen Carboxylgruppe mit einem ein- oder mehrwertigen Alkohol mit bis zu 6 Kohlenstoffatomen ganz oder teilweise verestert oder mit einem weiteren Melaminrest kondensiert sein kann und X mit Ausnahme von Ethylen ein geradkettiger oder verzweigter Alkylenrest mit 1 bis 12 Kohlenstoffatomen sein kann, der auch einen Cyclopentylen-, Cyclohexylen- oder Phenylenrest enthalten kann, oder 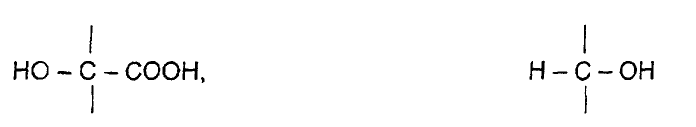
-[-CH₂-CH₂-O-]-ₙ - (CHOH)ₙ-(CH₂)ₘ-
oder 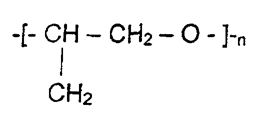 bedeuten kann, wobei m und n Werte zwischen 1 und 20 annehmen können und die anderen Reste R Wasserstoff, eine Alkylgruppe oder eine Acylgruppe mit bis zu 12 Kohlenstoffatomen sein können, sowie ihre Alkali-, Ammonium- oder Aminsalze.

2. Melamin-polycarbonsäureamid nach Anspruch 1, **dadurch gekennzeichnet, dass** alle Reste R Substituenten der Struktur II bedeuten.

3. Melamin-polycarbonsäureamid nach Ansprüchen 1 oder 2, **dadurch gekennzeichnet, dass** der Rest R sich von der Decandisäure oder von der Zitronensäure ableitet.

4. Melamin-polycarbonsäureamid nach Ansprüchen 1 oder 2, **dadurch gekennzeichnet, dass** eine Mischung verschiedener Reste der Struktur II mit dem Melamin verbunden sind.

5. Verfahren zur Herstellung von Melamin-polycarbonsäureamiden der Formel I, **dadurch gekennzeichnet, dass** man eine Schmelze einer Dicarbonsäure der Formel II
HOOC-X-COOH
oder seines Säurehalogenids, Säureanhydrids oder Esters, in der X eine der oben genannten Bedeutungen hat, in an sich bekannter Weise mit Melamin umsetzt, wobei ggf. im Verfahrensprodukt noch vorhandene freie Carboxylgruppen anschließend ganz oder teilweise mit einem ein- oder mehrwertigen Alkohol mit bis zu 6 Kohlenstoffatomen verestert, mit einem Mono- oder Dialkylamin oder mit einem Mono- oder Dialkanolamin mit jeweils bis zu 10 Kohlenstoffatomen unter Amidbindung umgesetzt werden können.

6. Korrosionsschutzmittel, **dadurch gekennzeichnet, dass** es ein Melamin-polycarbonsäureamid wie im Anspruch 1 beschrieben, enthält.

7. Korrosionsschutzmittel nach Anspruch 6, **dadurch gekennzeichnet, dass** es zusätzlich eine Monocarbonsäure mit 5 bis 12 Kohlenstoffatomen, vorzugsweise Isononansäure enthält.

## Claims

1. Melamin polycarboxylic amid with the following formula I: 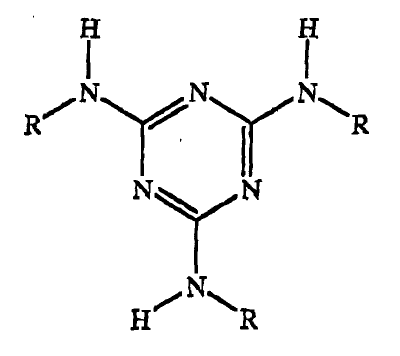 in which at least one of the rests R is a substitute with the structure II : 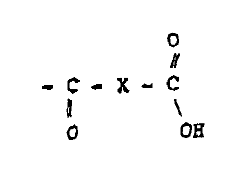 the carboxyl group of which can be partially or totally esterized with a mono- or polyvalent alcohol with up to 6 carbon atoms and can be condensed with another melamin rest and X can , except ethylen, be a straight or ramified alcyl rest with 1 to 12 carbon atoms, which can be contain a cyclopentylen, cyclohexylen or phenyl rest or can signifie: 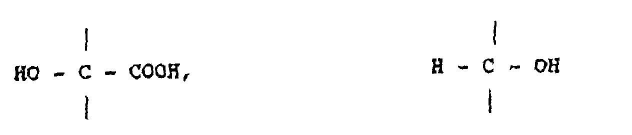
-[-CH₂ - CH₂ - 0 -]-ₙ, - (CHOH)ₙ - ( CH₂)ₘ - 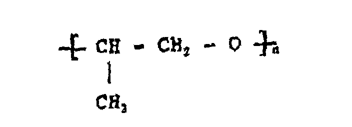
, whereas m and n can take values between 1 and 20 and the other rests R can be hydrogen, an alcyl group or an acyl group with up to 12 carbon atoms and their alkali, ammonium or amin salts.

2. Melamin polycarboxylic amid according to claim 1, caracterized in that all rests R signify substitutes of the structure II.

3. Melamin polycarboxylic amid according to claims 1 or 2, caracterized in that the rest R is deduced from sebacin acid or from citric acid.

4. Melamin polycarboxylic amid according to claims 1 or 2, caracterized in that the mixture of different rests of the structure II are linked with the melamin.

5. Method for the production of melamin polycarboxylic amid with the formula I, caracterized in that a melt of a bi-carbonic acid with the following formula II:
HOOC-X-COOH
or of its acid halogenids, acid hydride or esters, in which X has one of the above cited significations, is made to react in a known manner with melamin, whereas eventually existing free carboxyl groups afterwards can be esterized totally or partially with a mono or polyvalent alcohol with up to 6 carbon atoms or can be made to react under amid bond with a mono or bialcanomalin with up to 10 carbon atoms.

6. Corrosion inhibitor, caracterized in that it contains melamin polycarboxylic amid. as described in claim 1.

7. Corrosion inhibitor according to claim 6, caracterized in that it contains in addition a mono carbonic acid with 5 to 12 carbon atoms, advantageously isononan acid.

## Revendications

1. Amide d'acide polycarbonique de mélamine de formule I: 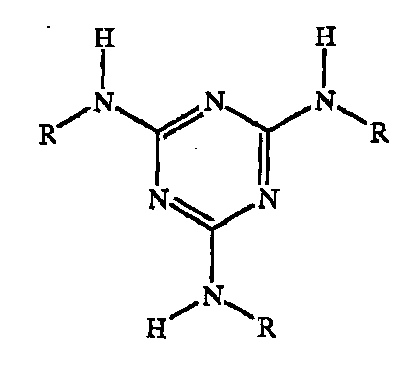 dans lequel au moins un des restes R est un substituant de structure II; 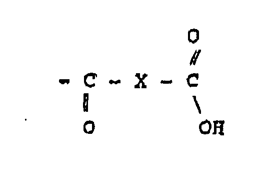 dont le groupe carboxyle est entièrement ou partiellement estérifié avec un alcool mono ou polyvalent de jusqu'à 6 atomes de carbone ; le groupe carboxyle peut être aussi condensé avec un autre reste de mélamine ;l'X, à l'exception de l'ethylène, peut être un reste alkyl, linéaire ou ramifié, avec un ou douze atomes de carbone et peut comprendre également un reste de cyclopentylène, de cyclohexylène ou de phényléne : 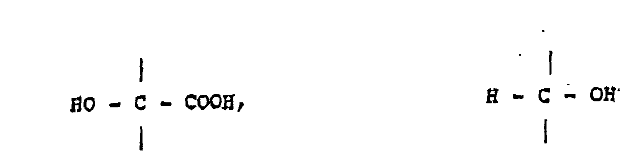
-[-CH₂ - CH₂ - O -]-ₙ, - (CHOH)ₙ - (CH₂)ₘ - 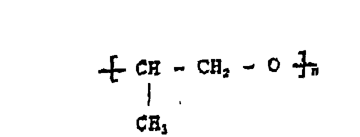
m et n pouvant prendre des valeurs entre 1 et 20 et les autres restes R pouvant être de l'hydrogène, un groupe alkyle ou un groupe acyle avec jusqu'à 12 atomes de carbone ainsi que leurs sels alcalins, ammoniacaux, et aminées.

2. Amide d'acide polycarbonique de mélamine selon la revendication1, **caractérisé en ce que** tous les restes R signifient des substituants de la structure II.

3. Amide d'acide polycarbonique de mélamine selon la revendication 1 ou 2, **caractérisé en ce que** le reste R est dérivé de l'acide sébacique ou de l'acide citrique.

4. Amide d'acide polycarbonique de mélamine selon la revendication 1 ou 2, **caractérisé en ce qu'**un mélange de différents restes de la structure II sont liés au melamine.

5. Procédé de fabrication d' amides d'acide polycarbonique de mélamine de formule I, **caractérisé en ce qu'**on fait réagir de façon connue une fonte d'acide dicarbonique de formule II:
HOOC-X-COOH
ou de sa halogénure d'acide, de son anhydride d'acide ou de son ester, dans laquelle formule X prend une des significations ci-dessus, avec de la mélamine, pendant que des groupes carboxyl encore présent dans le produit du procédé sont ensuite éventuellement estérifiés entièrement ou partiellement avec un alcool mono ou polyvalent de jusqu'à 6 atomes de carbone et qui peuvent réagir avec un mono ou dialcoylamine ou avec un mono ou dialcanomaline avec chaque fois jusqu'à 10 atomes de carbone avec une liaison amide.

6. Inhibiteur de corrosion, **caractérisé en ce qu'**il comprend un amide d'acide polycarbonique de mélamine selon la revendication 1.

7. Inhibiteur de corrosion selon la revendication 6, **caractérisé en ce qu'**il comprend en plus un acide monocarbonique de 5 à 10 atomes de carbone.
